# EUROPEAN PATENT SPECIFICATION

(11) **EP 4 157 155 B1**
(45) Date of publication and mention of the grant of the patent: **22.07.2026**
(21) Application number: 21818422.4
(22) Date of filing: 01.06.2021
(51) Int. Cl.: A61F 2/30

(54) **OSSEOINTEGRABLE DENTAL IMPLANTS OR SCREWS COMPRISING STRUCTURALLY POROUS SURFACE, PROCESS FOR PREPARING THE IMPLANTS AND SCREWS**
OSSEOINTEGRIERBARE ZAHNIMPLANTATE ODER SCHRAUBEN MIT STRUKTURELL PORÖSER OBERFLÄCHE, VERFAHREN ZUR HERSTELLUNG DER IMPLANTATE UND SCHRAUBEN.
IMPLANTS OU VIS DENTAIRES À OSTÉOINTÉGRATION COMPRENANT UNE SURFACE STRUCTURELLEMENT POREUSE, PROCÉDÉ DE PRÉPARATION DES IMPLANTS ET VIS

(30) Priority: 01.06.2020 BR 102020011004
(43) Date of publication of application: 05.04.2023
(73) Proprietor: M3 Health Indústria e Comércio de Produtos Médicos, Odontológicos e Correlatos S.A., 13212-213 Jundiaí (BR)
(72) Inventor: BLAY, Alberto, 01250-040 São Paulo (BR); WAGNER PINTO, Fabio, 13214-660 Jundiai (BR); AWAD SHIBLI, Jamil, 07115-230 Guarulhos (BR); TUNCHEL, Samy, 05012-000 São Paulo (BR); SASKA SPECIAN, Sybele, 06543-003 Santana de Parnaiba (BR)
(74) Representative: Robba, Pierpaolo
(86) International application number: PCT/BR2021/050235
(87) International publication number: WO 2021/243429

(56) References cited:
- WO-A1-2013/091085
- WO-A1-2018/234928
- US-A1- 2011 295 378
- US-A1- 2012 226 318
- US-A1- 2017 071 747
- US-A1- 2017 071 747
- US-A1- 2017 135 706

## Description

### FIELD OF THE INVENTION

**.** The present invention belongs to the technological fields of Dentistry and Medicine and refers to osseointegrated implants and screws with structurally porous surfaces prepared by an additive manufacturing process. Such implants are prepared in order to comprise modifications such as intrinsic porosity with controlled pore size and density and/or with reduced mass in specific regions, which confers several technical advantages.

**.** Furthermore, the present invention relates to the process of preparing said implants and/or screws with an optimized structure for accelerating osseointegration.

**.** Thus, the present invention refers to said implants and screws as carriers of proteins, drugs, stem cells, among other possibilities.

### BACKGROUND OF THE INVENTION

**.** Dental implants and screws, today, are the most modem and used to definitively rehabilitate teeth that have been lost. These are titanium pins/screws that are implanted in the bone and over them prostheses are cemented in composite resin, porcelain or zirconia (or both).

**.** Currently, osseointegrated implants and screws are commonly manufactured by machining or casting. In both cases, the resulting parts go through a machining process and subsequent surface treatment. With machining, the roughness is quite low (and this is usually the goal of the machining process).

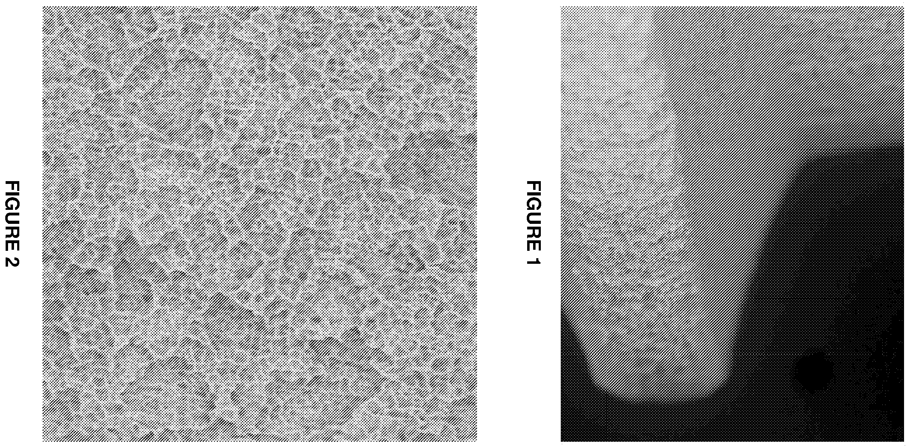

**.** Figures 1 and 2 illustrate details of examples of dental implant surface morphology from a traditional chemical process (acid attack) (photo zoomed 500 times and 2500 times respectively) (Source (http://www.cleanimplant.com/ date/)).

**.** It is known that the texturing obtained by these mechanical (blasting) or chemical (acid attack) processes is limited and quite random. Thus, there is no precise control of the desired surface configuration.

**.** The geometry of these components is merely oriented to mechanical resistance, in simple analysis of the clamping force or loading when the function of the planned implant.

**.** Screws are usually used to fix plaques, meshes or mesh to correct bone defects or to rebuild bone tissue due to trauma or tumors.

**.** The shape of the screw varies according to:
- Surgical technique applied;
- Location and, consequently, mechanical load applied to the screw.

**.** Most well-known products, currently on the market, are as follows:

| Medical Products with Trabecular Geometry Surface for use in Orthopedics and Traumatology | |
|---|---|
| Screw Products | |
| Item | Products |
| 1 | Monoaxial and polyaxial pedicle screw for spine fixation |
| 2 | Cannulated compression screw for fractures and deformities |
| 3 | Cannulated screw for fractures |
| 4 | Interference screw for ligament reconstruction |
| 5 | Herbert screw for fracture and deformity correction |
| 6 | Screw for fixing the odontoid |
| 7 | Acetabular Screw |

**.** In general, the surface roughness of a dental implant is about 1 µm (Rₐ, which is an arithmetic mean of the surface) or 10 µm (Rt, which corresponds to the greatest peak-valley distance). The roughness is obtained by blasting with spheres or abrasive particles, and subsequent acid attack (see Figure 2).

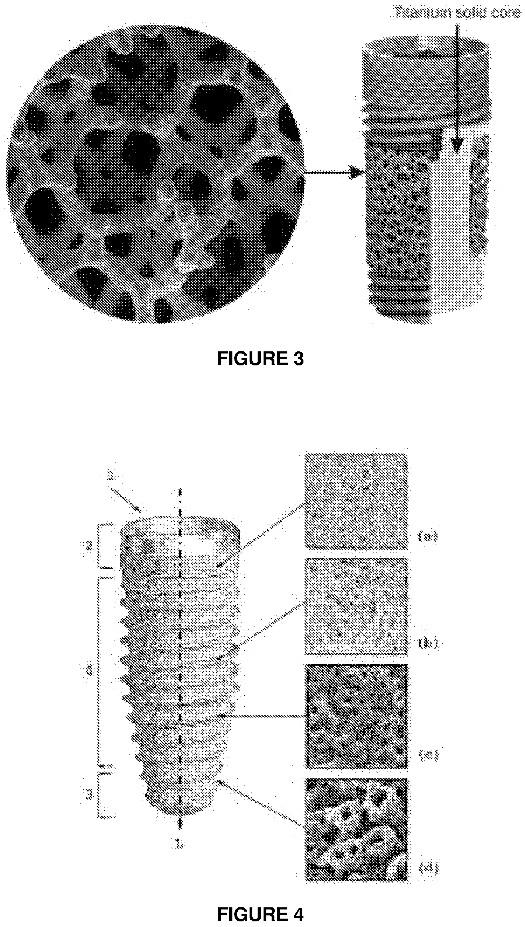

**.** Figures 3 and 4 illustrate screws prepared by conventional processes with limited control of the porosity or roughness of the part. Figure 4 illustrates a screw that has undergone chemical treatment to generate the surface microstructure.

**.** Thus, there is a technical limitation in the generation of implant roughness in addition to the little control of the result obtained by traditional processes.

**.** We highlight below some teachings of the state of the art that refer to the present subject:
**.** US 2017/071747 A1 refers to methods for designing and manufacturing customised orthopaedic implants, in particular patient-specific orthopaedic implants for insertion into a resected region of bone.

**.** WO 2013/091085 A1 refers to methods and systems for designing orthopaedic implants, and to implants with mechanically biocompatible cellular material.

**.** WO 2018/234928 A1 refers to a method of manufacturing implants with 3D printing techniques having a titanium-based structural body for bone integration, and implant devices made by the method.

**.** BR 112012031296 0 refers to implants that may include a base member, such as an acetabular shell or an add-on, which is configured for coupling with an add-on, a flange cup, a mounting member or any other adequate orthopedic display. Any of the implantable components can include one or more porous surfaces. The porous surface can be textured by projections that connect and extend from the surface. The sizes and concentration of the projections can be varied for specific applications to accommodate different patient anatomies and implants. A porous implant can also include one or more solid internal or external portions that stiffen the implant. It also describes that the porous implant can be created using 3D printing technology that uses powdered metal to "print" the modeled implant. In such approach, a foam can be created having a surface profile that includes protrusions and the profiled foam can then be filled in with the sprayed metal to create a porous microstructure with the profiled surface. A foam that does not contain the protrusions can also be used to create the porous microstructure with powdered metal and the desired surface profile with protrusions can then be stamped onto the surface of the porous metal implant.

**.** Document BR 10 2017 006922 2 reveals a tight bone implant, particularly an implant prototyped in titanium or zirconia, or cast in titanium, which turns to the health area, especially dentistry, specifically for rehabilitation treatment within the specialty of implant dentistry, surgery and prosthesis. The obtaining process, in one embodiment, requires a tomography of the patient's jaws, which is then subjected to a software for implant planning and subsequent machining of the mold, which can be done, for example, by machining on machine tools or on a 3D printer, to finally cast in titanium.

**.** Document KR 101635768 describes a method of manufacturing an artificial tooth using a three-dimensional (3D) printer. The 3D printer includes a raw material feed unit, a transfer unit, a print head, a work table and a control unit. The raw material feed unit is to feed a glass wire that serves as a raw material. The transfer unit is to transfer the glass wire fed from the raw material feed unit. The print head is to melt the glass wire transferred by the transfer unit and to discharge the glass wire through a nozzle.

**.** Document CN 207590772, on the other hand, describes a bone-type canine implant that comprises a self-threaded thread segment, a microporous thread segment and a thin thread segment arranged in order from top to bottom. The microporous segment of the structure simulates a spongy trabecular structure of human bone obtained by 3D printing.

**.** Document WO 2019/104853, on the other hand, describes a porous dental implant capable of degrading magnesium ions, comprising an implant-based cell printed by SLM technology of a pure titanium material, in which the implant-based cell has a porous structure and its surface is provided with a spherical hole or a groove. The groove, the spherical hole or the groove of the thread is filled with a degradable layer formed by the instantaneous bonding of the fusion of magnesium particles. SLM (selective laser
fusion) is an additive manufacturing method developed especially for 3D printing metal alloys. It creates parts, additively, by fusing particles of metal powder together in a complete melting process.

**.** Document CN 109172050 discloses a porous titanium sheet that can be filled with composites in a biological experiment and a method of preparing this sheet. It is said that the prototyping of the porous titanium sheet is done by 3D printing with process precision ≤0.025 mm.

**.** Finally, document CN 108236508 discloses a porous dental implant structure with self-expanding grooves with NiTi memory. It is said that the porous structures incorporated in the groove are distributed at the bottom of the root which is done by 3D printing with a memory effect.

**.** Therefore, in the state of the art, there is no solution equivalent to the one presented here in the present invention that combines technical differentials, economic advantages, safety and reliability of reproducibility in the manufacture of implantable medical devices with superficial porous microstructure interspersing the entire solid structure.

### OBJECTIVES OF THE INVENTION

**.** Thus, it is an objective of the present invention to provide osseointegrated implants and screws for dental and medical use (prostheses).

**.** It is an objective of the present invention to provide osseointegrated implants and screws with surface structure morphologically with controlled and rougher geometry.

**.** It is an objective of the present invention to provide osseointegrated implants and screws prepared by means of an additive manufacturing process (3D printing).

**.** It is another objective of the present invention to provide osseointegrated implants and screws prepared by means of a process that allows control of the roughness distribution as well as the pore size and morphology.

**.** It is another objective of the present invention to provide osseointegrated implants whose surface presents controlled porosity many times greater when compared to implants prepared by traditional processes.

**.** It is another object of the present invention to provide implants whose surface is plainly distinct, with regions where the roughness is altered according to the stipulated requests or requirements.

**.** It is also another objective of the present invention to provide implants with better integration into the body, due to the microstructure of the surface.

**.** It is, yet, another objective of the present invention to provide implants that provide faster rehabilitation of the patient.

**.** It is, yet, another objective of the present invention to provide implants whose preparation process requires less use of material (if porous, lighter, less material), thus less quantity of product to be implantable (reduction of foreign body to the organism), without compromise the mechanical property requirements for the performance of the intended function.

**.** Furthermore, it is an objective of the present invention to provide osseointegrable implants used as carriers.

**.** It is, yet, another objective of the present invention to provide implants with specific texture that attends the identified need of the place where the implant or screw will be fixed.

### SUMMARY OF THE INVENTION

**.** The present invention achieves these and other objectives through a process for preparation of a dental implant or screw having a trabecular region and a solid region, comprising the following steps:
a) preliminary preparation of a dental implant or screw (endo-osseous implantology) or selection of available implant or screw model;
b) analysis of the intended location of the implant or screw, for example, morphological analysis by imaging tests such as computed tomography of the indicated location;
e) obtaining patient data to verify integration needs;
d) analysis of the loading of the implant or screw in order to determine an optimized topology of the implant or screw;
e) computer model of a texture comprising designing of a porous surface comprising geometry and pore size, wall thicknesses and other characteristics of the implant or screw surface, wherein the textured surface is limited to the trabecular region and is determined geometrically by the shape of the implant or screw; and
f) reproduction of the texture in the implant or screw in a controlled manner using the additive manufacturing technique;
wherein after the additive manufacturing said process comprises the following additional steps:
g) heat treatment,
h) sintering,
i) machining,
j) cleaning,
k) anodizing,
l) laser marking,
m) quality control,
n) final cleaning,
o) packaging,
p) labeling, and
q) sterilization.

**.** The present invention achieves these and other objectives by means of an implant or screw obtained by the above process.

### BRIEF DESCRIPTION OF THE DRAWINGS

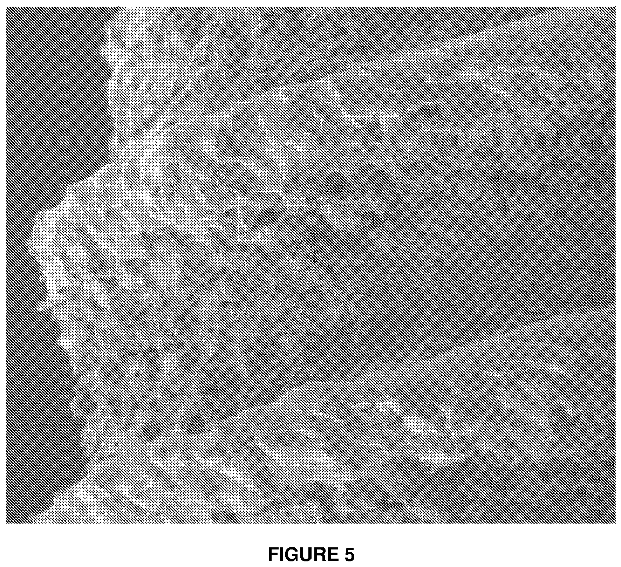

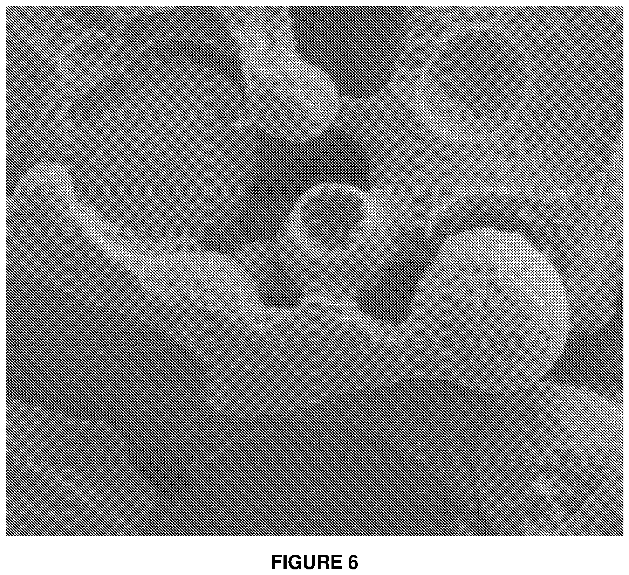

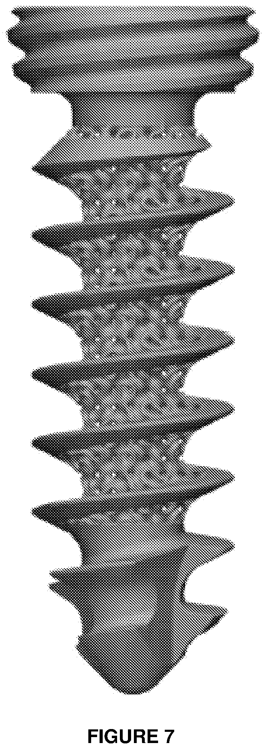

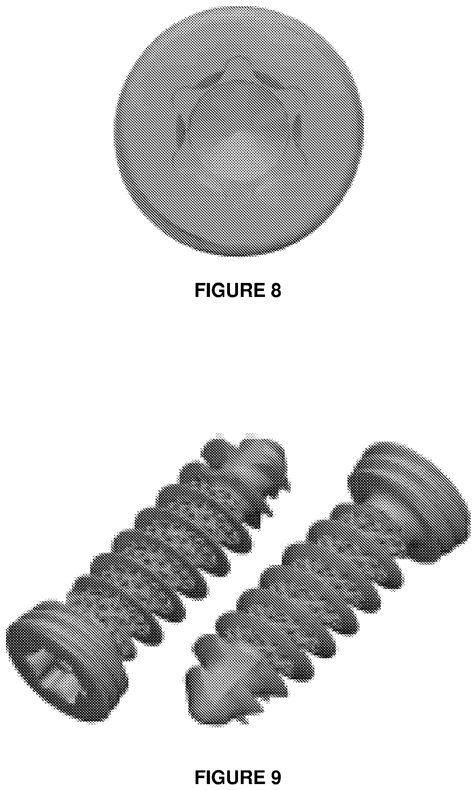

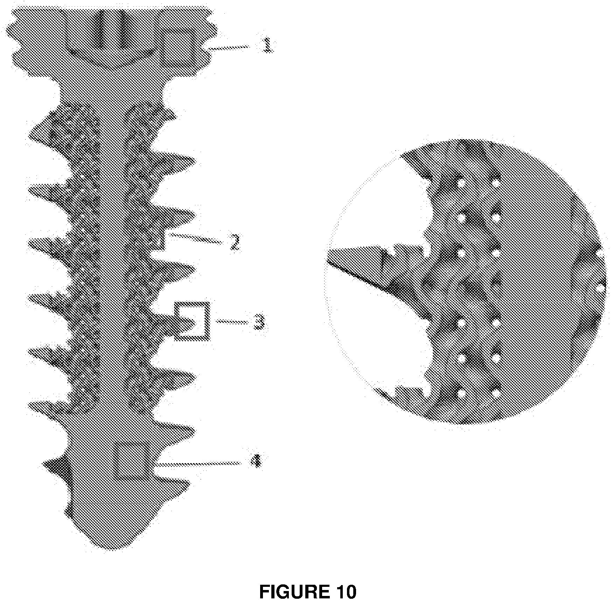

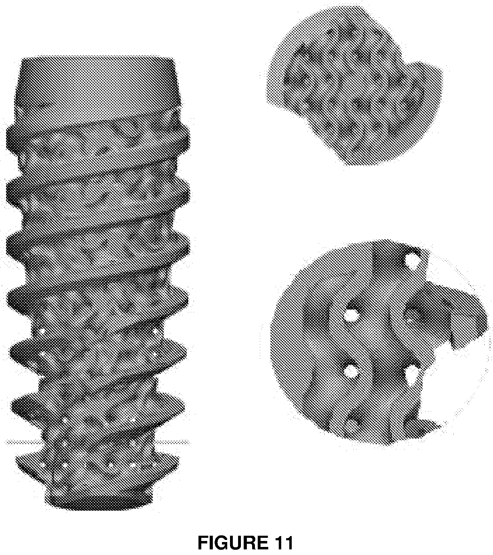

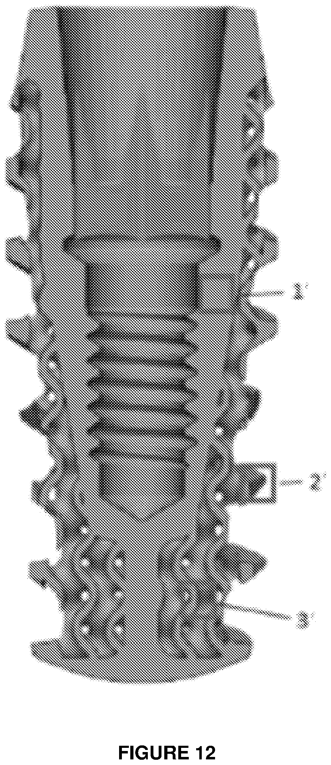

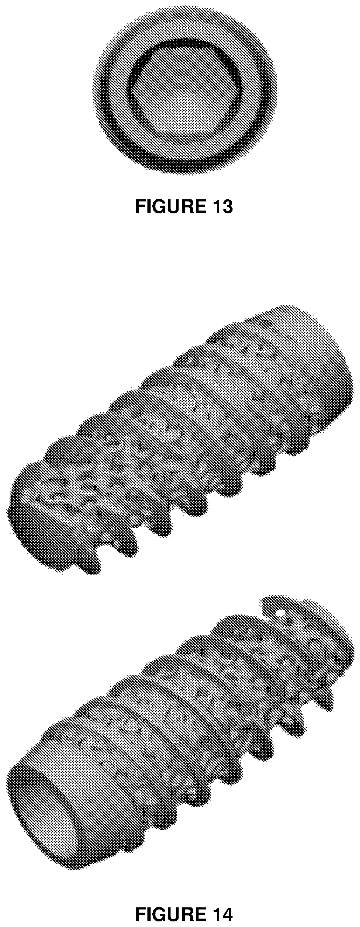

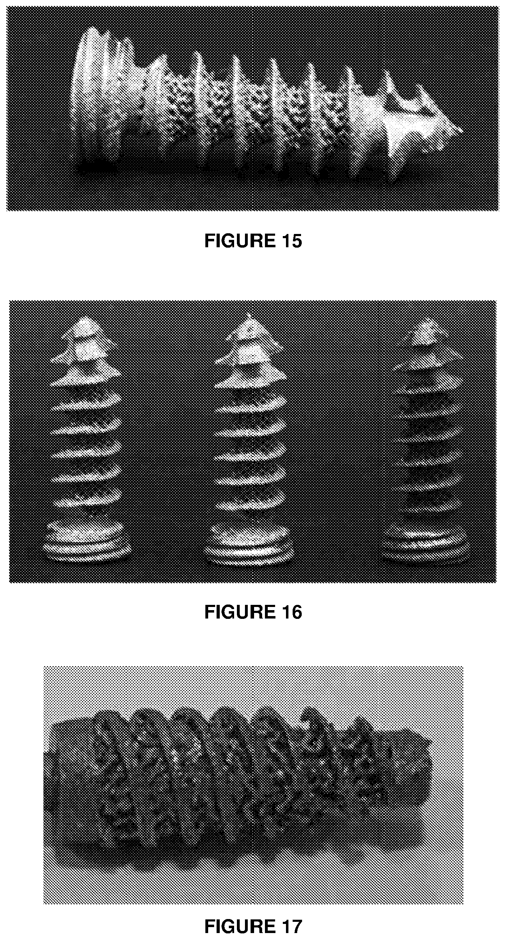

**.** The present invention will be described on the basis of the drawings attached hereafter, which illustrate:
**.** Figure 1 illustrates a detail of a dental implant, of the state of the art, prepared by a traditional process;
**.** Figure 2 illustrates a detail of a dental implant, of the state of the art, prepared by a traditional process;
**.** Figure 3 shows a detail of a dental screw, from the state of the art, prepared by a traditional process;
**.** Figure 4 shows a detail of a dental screw, from the state of the art, prepared by a traditional process;
**.** Figure 5 illustrates a detail of the surface of a dental implant manufactured using 3D printing technology, but without the microstructure of complex geometry;
**.** Figure 6 illustrates the detail of the surface morphology of a dental implant manufactured via 3D printing technology;
**.** Figure 7 illustrates a side view of a dental screw, object of the present invention, where it is possible to observe the architecture of the complex geometry and with controlled pore size in its structure;
**.** Figure 8 illustrates a top view of a dental screw, the object of the present invention;
**.** Figure 9 illustrates two perspective views of a dental screw, the object of the present invention;
**.** Figure 10 illustrates longitudinal section of a dental screw, object of the present invention, where it is possible to observe the architecture of the complex geometry and with controlled pore size in its structure;
**.** Figure 11 illustrates side view and expanded details of a dental implant, object of the present invention, where it is possible to observe the architecture of the complex geometry and with controlled pore size in its structure;
**.** Figure 12 illustrates longitudinal section of a dental implant, object of the present invention;
**.** Figure 13 illustrates a top view of a dental implant, object of the present invention;
**.** Figure 14 illustrates perspective views of a dental implant, the object of the present invention;
**.** Figure 15 is a photo of the dental screw, object of the present invention;
**.** Figure 16 is a photo of colored versions of the dental screw, object of the present invention;
**.** Figure 17 is a photo of the dental implant, the object of the present invention.

### DETAILED DESCRIPTION OF THE INVENTION

**.** The present invention relates to osseointegrated implants and screws prepared from additive manufacturing (commonly known with 3D printing). With this process, it is possible to influence the texture (microstructure) of the surface and the mechanical structure of the implantable medical device. It is noteworthy that other known manufacturing processes are not capable of this feat.

**.** Through this process, it is possible to control the roughness morphology and even the complete structure of the implant (for example, ceasing to be massive and becoming porous in regions of less mechanical stress) as can be seen from Figures 5 and 6.

**.** The impossibility of influencing the texture limits the use of the processes traditionally used in the preparation of texture with geometry and controlled pores of dental and medical implants. Conventional processes of obtaining and endo-osseous screws and implants do not allow the control of the texture of dental and medical implants. With the present invention, the texture achieved allows, still to serve as a reserve for biomodulators, materials that would be stored in specific positions and that favor the growth of specific cells, or even the inhibition of others (for example, bactericidal/bacteriostatic in the upper part - close to the gingiva - and osteoblast stimulators in the region of the apex - tip - of the implant. This type of selective "carrying" for biomodulators, or even greater geometric affinity, cannot be achieved with the conventional process and can be easily verified with the cell growth analysis process.

**.** Thus, the implant, object of the present invention, comprises a much rougher surface when compared to implants prepared by traditional processes.

**.** Further, the process of the present invention allows to result in an implant with a much rougher surface as well as to program textures on the surface with geometric freedom unattainable in any other process. Thus, there are at least three advantages with the use of the process of the present invention when compared to the state of the art:
- A rougher surface is more interesting to osseointegration and cannot be obtained by conventional manufacturing processes;
- Process allows, in addition to the naturally rougher surface, to generate superficial microstructure that provide even better bone integration of the implant and screw (implantable medical devices);
- Textures can be used to carry (drug release) - peptides, proteins growth factors, antimicrobial and phototherapeutic drugs and stem cells as well as different amounts of biostimulators or antimicrobials (chemical agents), selective transport, which encourage growth or fighting certain cells.

**.** It is important to note that the human being has very different cellular structures in human bone. While the outermost layer (cortical) of the surface is dense and of greater hardness, the innermost region of the bone (medullary) has a more porous structure.

**.** With the control of the texture of the implant (, it is possible to design textures that meet the specific need of the place where the implant will be used.

**.** Thus, in addition to producing line products with these characteristics, with texture control, customized products can be manufactured for the patient, or by known region of the body. In a preferred embodiment, for the mandible, some standard solutions are prepared with, for example, 60% of the implant with a first texture and 40% with a second texture.

**.** Further, the possibility of storing different amounts of molecules (chemical agents) allows to encourage greater growth of certain cell types in different regions of the implant, or even the fight against microorganisms in situations of infection.

**.** Figure 9 illustrates an example of a dental screw in which the reduction in mass of said screw is 26% in relation to the screw of similar geometry currently available on the market, but without the external part with modified microstructure.

**.** Figures 7 and 10 illustrate an example of orthopedic screw composition with structure modification.

**.** Figure 7 illustrates a view of the screw with porous microarchitecture and complex geometry.

**.** Figure 10 illustrates a longitudinal section of the screw with emphasis on:
- first and fourth regions 1 and 4, where the density is the maximum possible because it is a region with high loads when using and inserting the screw in bone tissues;
- a second region 2 where there is a reduction in density in a planned way, aiming at increasing the available area to favor the growth of bone tissue within this controlled porous microarchitecture;
   - a third region 3 that has a structural function and undergoes frictional loads on insertion into bone tissue, being necessarily massive.

**.** Figures 11 and 12 show an example of dental implant composition with structure modification.

**.** Figure 11 illustrates a view of the implant with porous microarchitecture and complex geometry.

**.** Figure 12 illustrates a longitudinal section of the implant with emphasis on:
- a first 1' region, where the density is the maximum possible because it is a region with high loads when using the implant for its purpose (dental rehabilitation);
- a second region 2' that has a structural function and suffers frictional loads, being necessarily massive;
- a third 3' region where there is a reduction in density in a planned way, aiming at increasing the available area to favor the growth of bone tissue within this controlled porous microarchitecture.

**.** It should be noted that the mass reduction data between 26 and 30% presented above are exemplary. Structural optimizations can lead to very different reductions, always considering the optimization that the product is aimed for in order to fulfill its function.

**.** Figure 14 illustrates an example of a solution, where the innermost part of the screw is kept solid while another (outermost) part of the implant has had its structure changed to a porous micro-architecture with complex geometry by adopting a gyroid structure. In the region in question, the density of the implant became 36% of the solid part in the trabeculated region. The total reduction in the mass of the implant with the adoption of this porous section is 30% in relation to the same massive model.

**.** The process of the present invention comprises the following steps:
- Model the texture in a specific way on a computer; and
- Reproduce the texture on an implant or prosthesis in a controlled manner using the additive manufacturing technique.

**.** In other words, the process of preparing an osseointegrated dental implant or screw (implantable medical device) of the present invention comprises the following steps:
a) preliminary preparation of a dental implant or screw (endo- osseous implantology) or selection of implant or screw available;
b) analysis of the intended location of the product, for example, morphological analysis by imaging tests such as computed tomography of the indicated location;
e) obtaining patient data to verify integration needs;
d) analysis of the loading of the implant or screw in order to determine an optimized topology of the implant;
e) computer model of a texture comprising designing of the porous surface comprising geometry and pore size, wall thicknesses and other characteristics of the implant or screw surface; and
f) reproduction of the texture in the implant or screw in a controlled manner using the additive manufacturing technique.

**.** One embodiment of the production process of the present invention comprises the following steps:
a) Design involves topological optimization aiming at the transmission of mechanical efforts. Here, the characteristics and mechanical properties of the different raw materials are considered (metals, polymers or ceramics including titanium and its alloys, nitinol alloys - NiTi, resorbable metal alloys based on magnesium, manganese and zinc, bioceramics such as: hydroxyapatite, beta -tricalcium phosphate and zirconia);
b) Subsequently, the trabecular/porous region is projected, limited geometrically by the shape of the screw. The porosity/geometry of the porous region is also projected considering the possibility of coating with carriers of chemical agents and/or biomolecules;
c) Once the complete design of the component is established, digital processing is then carried out to prepare the additive manufacturing process;
d) The physical component is obtained by the additive manufacturing process. The process is chosen according to the raw material adopted;
e) After the additive manufacturing process, there will be, according to the raw material, the subsequent process of heat treatment (when applicable, for metallic and bioceramics only), sintering (when applicable, for bioceramics only), machining (when applicable, for metal only), cleaning, anodizing (when applicable, for metallic ones only), laser marking (when applicable, according to the total dimension of the component), quality control, final cleaning, packaging, labeling and sterilization.

**.** With the use of the chemical and mechanical processes currently used, there is no possibility of obtaining surfaces with this structural architecture like those obtained by the process of the present invention. This advantage is a great differential because:
- Different people have different bone structures;
- Different body regions have very different bone structures;
- The process of the present invention allows more precise control of the geometry and size of the interconnected pores to apply as texture, and direct comparison to a model, for example, CAD model (design and computer) between what was designed and what was obtained;
- Stimulators or cell growth inhibitors can be added to the implant in specific positions and in controlled quantities, called selective carriage;
- It becomes possible to model the texture in a specific way on a computer and reproduce it in a controlled way using the process of the present invention. The more determined control of the geometry stands out than with any other process.

**.** Therefore, the process of the present invention and the products generated by said process (screws and implants with designed structure) offer additional differentials:
- Regarding the function of the screws: better/faster meeting the patient's need regarding the reestablishment/rehabilitation of the function and/or the aesthetic action;
- As for the design made specifically for the projected load: Topology aimed at minimizing the material of the implant/screw under the condition of support of the mechanical efforts applied to the component (topological optimization) with 25 to 40% reduction of raw material;
- As for the control for carrying cells/drugs: topology aiming at structuring the position and number of carriers of molecules along each component according to the application and purpose;
- Regarding productivity and financial viability: technical/financial viability of the production of items with great geometric complexity and optimized according to the application;
- Regarding the possibility of different raw materials for the pieces: design method allows diversity of materials in the product with very similar (but not the same) production principles, differently from traditional production processes that have clear limitations regarding the diversity of raw materials.

**.** It is noteworthy that the design and production process observe geometric characteristics that cannot be obtained by means of subtractive manufacturing, but only by the additive manufacturing process. This characteristic is essential and genuine to the present invention.

**.** The understanding of the mechanical load on the components is a limiting factor of the implant/screw area with a trabecular region (2). It is understood here that the transmission of forces on the implant depends on whether it is massive. Thus, if the loads are very high and the screw has a limited diameter, it is possible that the region with trabeculated structure is limited.

**.** The present invention has the following main features and components:
- The structure combines solid portions (aiming at mechanical stability) and porous screw/implant (aiming to increase the surface area of the implant, increase the area of bone integration, possibility of coating with cellular carrier agent);
- The design considers the load applied in each region of the body, thus generating families of screws/implants with known characteristics;
- The porous region allows for the accommodation of cell growth carriers;
- The quantity and location of the cell carrier agents are controlled by the geometric shape of the pores induced in the porous section of the piece;
- The manufacturing process allows the production of a range of implant/screw geometries within shorter production times than by conventional manufacturing processes.

**.** Below is a detail of the operation of the present invention:
- The function of the screw/implant is to reestablish the patient's motor/masticatory/aesthetic function;
- The increase in area caused by the geometry of the macro and microstructure (texturing) of the surface allows bone tissue to occupy these places and increase tissue integration;
- Through component loading analysis (biomechanical analysis and finite element simulation), the optimized screw/implant topology is determined;
- Once the solid topology has been determined, the porous process is designed, where the pore size, wall thicknesses and other characteristics of the piece are described.
- The porous region allows accommodation of carrier agents in such a way that different agents are deposited in different positions of the trabeculated region.

**.** It is observed that the porous fraction of the implant can be limited due to the need to maintain the region/solid fraction of the implant due to the mechanical loads applied. Also, the reduction of the solid region (1, 3, 4) limits the capacity of the support component to situations of possible overload.

**.** And, due to the characteristic of neoformation of bone tissue, there is an ideal pore size of 0.3mm in diameter that can be designed on the part for the functionalization of cell growth having the maximum limit dependent on each part to be produced because it is directly related reduction of mechanical resistance.

**.** It is important to note that the loads vary according to the position of the screw used in the body.

**.** Also, the lengths and diameters of the screws/implants are given according to minimize the invasion in the organism and guarantee the intended function.

**.** The parameters of the trabecular region (sometimes denser, sometimes more porous, with control of geometric characteristics in the design) allows to design/accommodate different amounts of cell carrier agents. It is also possible to change the trabecular geometry between the regions of the implant/screw.

**.** Figures 7 to 14 illustrate views of the screw and the implant, objects of the present invention. In these figures, it is possible to verify the distribution and density of the pores present on the surface and structure of preferred embodiments of the present invention.

**.** Figures 15, 16 and 17 are pictures of the screw and the implant, objects of the present invention.

**.** In preferred embodiments of the present invention, the following screw models for orthopedic purposes are used:
- Monoaxial and polyaxial pedicle screw for fixing the spine;
- Cannulated compression screw for fractures and deformities;
- Cannulated screw for fractures;
- Interference screw for ligament reconstruction;
- Herbert type screw to correct fractures and deformities;
- Screw for fixing the odontoid;
- Acetabular screw.

**.** The present invention has numerous technical and economic advantages when compared to the state of the art, some of which are listed below:
✔ Reduction of material by implantable medical device - reduction of the possibility of rejection of the implant (foreign body reaction);
✔ Greater growth of adjacent bone tissue within the porous microstructure, resulting in better adherence of the implant to the body;
✔ Design of parts with mechanical characteristics closer to those of the bone tissue where it will be applied, especially considering the elasticity of the part;
✔ Better adaptation of the tissues adjacent to the implants/screws;
✔ Add functions and stimulators in controlled positions and quantities in the implants;
✔ Possibility of the implant being a carrier of drugs and/or biomolecules distributed in the porosities designed on the implant. Thus, there is the additional function of treating a specific site with the use of the aforementioned bioactive molecule or even acting in a combined manner on the site;
✔ Possibility of the implant being a carrier of stem cells, these distributed in the porosities designed on the implant, causing better fixation and healing of the implant.

. Having described an example of a preferred embodiment of the present invention, it should be understood that the scope of the present invention is defined by the appended claims.

## Claims

1. Method for preparation of dental implant or screw, said implant or screw having a trabecular region (2) and a solid region (1, 3, 4), comprising the following steps:
a) preliminary preparation of a dental implant or screw model or selection of an available implant or screw model;
b) analysis of the intended location of the implant or screw, for example, morphological analysis by imaging tests such as computed tomography of the indicated location;
c) obtaining patient data to verify integration needs;
d) analysis of the loading of the implant or screw in order to determine an optimized implant or screw topology;
e) computer model of a texture comprising designing of a porous surface comprising geometry and pore size, wall thicknesses and other characteristics of the implant or screw surface, wherein the textured surface is limited to the trabecular region (2) and is determined geometrically by the shape of the implant or screw; and
f) reproduction of the texture in the implant or screw in a controlled manner using the additive manufacturing technique;
wherein after the additive manufacturing said process comprises the following additional steps:
g) heat treatment,
h) sintering,
i) machining,
j) cleaning,
k) anodizing,
l) laser marking,
m) quality control,
n) final cleaning,
o) packaging,
p) labeling, and
q) sterilization.

2. Method, according to claim 1, **characterized by** the implant or screw loading analysis of step d) is carried out by means of biomechanical analysis or numerical simulation by the finite element method.

3. Method, according to claim 2, **characterized by** the pore diameter is equal to or greater than 0.3mm.

4. Method, according to claim 1, **characterized by** the material used in step f) is selected from the group consisting of metals, polymers or ceramics including resorbable or non-resorbable biocompatible polymers, titanium and its alloys, resorbable metal alloys, other metal alloys such as NiTi (nitinol) and bioceramics.

5. Dental implant, **characterized by** being obtained through a process as defined in any one of claims 1 to 4.

6. Dental implant according to claim 5, **characterized by** being a drug carrier, the drug being selected from the group consisting of peptides, growth factor proteins, antimicrobial and phototherapeutic drugs, stem cells and biostimulator.

7. Dental screw, **characterized by** being obtainable through a process as defined in any one of claims 1 to 4.

8. Dental or orthopedic screw according to claim 7, **characterized by** being a drug carrier, the drug being selected from the group consisting of peptides, growth factor proteins, antimicrobial and phototherapeutic drugs, stem cells and biostimulator.

## Patentansprüche

1. Verfahren zur Herstellung eines Zahnimplantats oder einer Zahnschraube, wobei das Implantat oder die Schraube einen trabekulären Bereich (2) und einen festen Bereich (1, 3, 4) aufweist, umfassend die folgenden Schritte:
a) Vorbereiten eines Modells von Zahnimplantat oder Zahnschraube oder Wählen eines verfügbaren Modells von Implantat oder Schraube;
b) Analysieren der vorgesehenen Position des Implantats oder der Schraube, beispielweise, morphologische Analyse mittels bildgebender Untersuchungen wie Computertomographie der angegebenen Position;
c) Erhalten von Patientendaten zur Überprüfung von Integrationsanforderungen;
d) Analysieren der Implantat- oder Schraubenbelastung zur Bestimmung einer optimierten Implantat- oder Schraubentopologie;
e) computergestütztes Modellieren einer Textur umfassend das Gestalten einer porösen Oberfläche, das Geometrie und Porengröße, Wandstärken und andere Merkmale der Implantat- oder Schraubenoberfläche umfasst, wobei die texturierte Oberfläche auf den trabekulären Bereich (2) beschränkt ist und geometrisch durch die Form des Implantats oder der Schraube bestimmt ist; und
f) Reproduzieren der Textur in dem Implantat oder in der Schraube auf kontrollierte Weise mithilfe der Technik der additiven Fertigung;
wobei, nach der additiven Fertigung, das Verfahren die folgenden zusätzlichen Schritte umfasst:
g) Wärmebehandlung,
h) Sinterung,
i) Zerspanung,
j) Reinigung,
k) Anodisierung,
l) Laser-Markierung,
m) Qualitätslenkung,
n) Endreinigung,
o) Verpackung,
p) Kennzeichnung, und
q) Sterilisierung.

2. Verfahren nach Anspruch 1, **dadurch gekennzeichnet, dass** das Analysieren der Implantat- oder Schraubenbelastung im Schritt d) durch biomechanische Analyse oder numerische Simulation mittels der Finite-Elemente-Methode durchgeführt wird.

3. Verfahren nach Anspruch 2, **dadurch gekennzeichnet, dass** die Porengröße gleich oder größer als 0,3 mm ist.

4. Verfahren nach Anspruch 1, **dadurch gekennzeichnet, dass** das im Schritt f) verwendete Material aus der Gruppe ausgewählt ist, bestehend aus Metallen, Polymeren oder Keramiken einschließlich resorbierbarer oder nicht resorbierbarer biokompatibler Polymere, Titan und seine Legierungen, resorbierbare Metalllegierungen, andere Metalllegierungen wie NiTi (Nitinol) und Biokeramiken.

5. Zahnimplantat, **dadurch gekennzeichnet, dass** es durch ein Verfahren hergestellt wird, wie es in einem der Ansprüche 1 bis 4 definiert ist.

6. Zahnimplantat nach Anspruch 5, **dadurch gekennzeichnet, dass** es ein Medikamententräger ist, wobei das Medikament aus der Gruppe ausgewählt ist, bestehend aus Peptiden, Wachstumsfaktorproteinen, antimikrobiellen und phototherapeutischen Arzneimitteln, Stammzellen und Biostimulatoren.

7. Zahnschraube, **dadurch gekennzeichnet, dass** sie durch ein Verfahren hergestellt werden kann, wie es in einem der Ansprüche 1 bis 4 definiert ist.

8. Zahnschraube oder orthopädische Schraube nach Anspruch 7, **dadurch gekennzeichnet, dass** sie ein Medikamententräger ist, wobei das Medikament aus der Gruppe ausgewählt ist, bestehend aus Peptiden, Wachstumsfaktorproteinen, antimikrobiellen und phototherapeutischen Arzneimitteln, Stammzellen und Biostimulatoren.

## Revendications

1. Procédé pour préparer un implant dentaire ou une vis dentaire, cet implant ou cette vis comportant une région trabéculaire (2) et une région solide (1, 3, 4), dans lequel le procédé comporte les étapes suivantes:
a) préparation préliminaire d'un modèle d'implant dentaire ou de vis dentaire, ou sélection d'un modèle disponible d'implant dentaire ou de vis dentaire;
b) analyse de l'emplacement prévu de l'implant ou de la vis, par exemple, analyse morphologique par des examens d'imagerie tels que la tomodensitométrie de l'emplacement indiqué;
c) obtention de données du patient afin de vérifier les besoins d'intégration ;
d) analyse de la charge de l'implant ou de la vis afin de déterminer une topologie optimisée de l'implant ou de la vis;
e) modélisation par ordinateur d'une texture comprenant la conception d'une surface poreuse comprenant la géométrie et la taille des pores, l'épaisseur de paroi et d'autres caractéristiques de la surface de l'implant ou de la vis, dans laquelle la surface texturée est limitée à la région trabéculaire (2) et elle est déterminée géométriquement par la forme de l'implant ou de la vis; et
f) reproduction de la texture dans l'implant ou dans la vis de manière contrôlée à l'aide de la technique de fabrication additive;
dans lequel, après la fabrication additive, ce procédé comprend les étapes supplémentaires suivantes :
g) traitement thermique,
h) frittage,
i) usinage,
j) nettoyage,
k) anodisation,
l) marquage laser,
m) contrôle qualité,
n) nettoyage final,
o) conditionnement,
p) étiquetage, et
q) stérilisation.

2. Procédé selon la revendication 1, **caractérisé en ce que** l'analyse de la charge de l'implant ou de la vis dans l'étape d) est réalisée au moyen d'une analyse biomécanique ou d'une simulation numérique par la méthode des éléments finis.

3. Procédé selon la revendication 2, **caractérisé en ce que** le diamètre des pores est égal ou supérieur à 0,3 mm.

4. Procédé, selon la revendication 1, **caractérisé en ce que** le matériau utilisé dans l'étape f) est choisi parmi le groupe constitué par métaux, polymères ou céramiques, y compris des polymères biocompatibles résorbables ou non résorbables, le titane et ses alliages, des alliages métalliques résorbables, d'autres alliages métalliques tels que le NiTi (nitinol) et des biocéramiques.

5. Implant dentaire, **caractérisé en ce qu'**il est obtenu par un procédé tel que défini dans l'une quelconque des revendications 1 à 4.

6. Implant dentaire selon la revendication 5, **caractérisé en ce qu'**il est un vecteur de médicament, le médicament étant choisi parmi le groupe constitué par peptides, protéines de facteurs de croissance, médicaments antimicrobiens et photothérapeutiques, cellules souches et biostimulateurs.

7. Vis dentaire, **caractérisée en ce qu'**elle peut être obtenue par un procédé tel que défini dans l'une quelconque des revendications 1 à 4.

8. Vis dentaire ou orthopédique selon la revendication 7, **caractérisée en ce qu'**elle est un vecteur de médicament, le médicament étant choisi parmi le groupe constitué par peptides, protéines de facteurs de croissance, médicaments antimicrobiens et photothérapeutiques, cellules souches et biostimulateurs.
